# EUROPEAN PATENT APPLICATION

(11) **EP 4 454 630 A1**
(43) Date of publication of application: **30.10.2024**
(21) Application number: 22911312.1
(22) Date of filing: 21.12.2022
(51) Int. Cl.: A61K 6/887, A61K 6/836

(54) **DENTAL CURABLE COMPOSITION**

(30) Priority: 21.12.2021 JP 2021207552
(71) Applicant: Kuraray Noritake Dental Inc., Kurashiki-shi, Okayama 710-0801 (JP)
(72) Inventor: SUZUKI, Kenji, Tainai-shi, Niigata 959-2653 (JP)
(74) Representative: D Young & Co LLP
(86) International application number: PCT/JP2022/047232
(87) International publication number: WO 2023/120610

(57) **Abstract**

The present invention relates to a dental curable composition containing a (meth)acrylic based compound (A) having two or more (meth)acryloyl groups in one molecule, a particular mono(meth)acrylate ester compound (B), a chemical polymerization initiator (C), a polymerization accelerator (D), and a filler (E), being separately packaged in a first material containing the chemical polymerization initiator (C) and a second material containing the polymerization accelerator (D); a dental filling and restorative material containing the dental curable composition; a dental cement containing the dental curable composition; and a dental abutment building material containing the dental curable composition.

## Description

### Technical Field

The present invention relates to a two-component type dental curable composition that is suitable as a dental filling and restorative material, a dental cement, and a dental abutment building material, and particularly as a dental filling and restorative material used for a large cavity.

### Background Art

A dental composite resin constituted mainly by a polymerizable monomer, a filler, and a polymerization initiator has become the most frequently used dental material for restoring a defective part of a tooth or dental caries, or for bonding dental prostheses. Examples of the dental material include a dental filling and restorative material, a dental cement, and a dental abutment building material. Improvements of the dental composite resin are being demanded in the following points. Specifically, examples of the improvements for the cured product thereof include the enhancement of the mechanical strength and the reduction of the polymerization shrinkage in curing.

There is a strong demand of the reduction of the polymerization shrinkage as much as possible since a contraction gap is caused thereby through peeling of the dental composite resin from the adhesion surface. The occurrence of a contraction gap becomes a factor of secondary caries, pulpal irritation, coloration, drop off of the prosthesis, and the like.

The following techniques have been known as a measure for reducing the polymerization shrinkage. For example, PTL 1 describes a dental restorative curable composition containing a (meth)acrylate ester compound having two or more (meth)acryloyloxy groups in one molecule, a particular mono(meth)acrylate ester compound, an inorganic filler having a particular primary particle diameter, and a polymerization initiator.

### Citation List

### Patent Literature

PTL 1: WO 2020/218446

### Summary of Invention

### Technical Problem

However, as a result of the investigations by the present inventor, it has been found that the dental curable composition described in PTL 1 has a reduced polymerization shrinkage stress, but has room for improvement in the application thereof particularly to filling and restoration of a large cavity, abutment building, and adhesion of a prosthesis having a large adhesion surface.

Under the circumstances, an object of the present invention is to provide a dental curable composition that has a particularly small polymerization shrinkage stress and is excellent in the mechanical strength of the cured product.

### Solution to Problem

The present invention encompasses the following inventions.
[1] A dental curable composition containing a (meth)acrylic based compound (A) having two or more (meth)acryloyl groups in one molecule, a mono(meth)acrylate ester compound (B), a chemical polymerization initiator (C), a polymerization accelerator (D), and a filler (E), the mono(meth)acrylate ester compound (B) containing at least one kind selected from the group consisting of a mono(meth)acrylate ester compound (b-I) represented by the following general formula (I) and a mono(meth)acrylate ester compound (b-II) represented by the following general formula (II), the dental curable composition being separately packaged in a first material containing the chemical polymerization initiator (C) and a second material containing the polymerization accelerator (D): 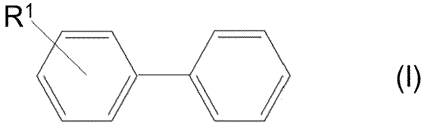 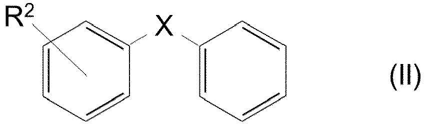 in which in the general formulae (I) and (II), R¹ and R² each independently represent a group represented by the following general formula (i) or a group represented by the following general formula (ii), and X represents a divalent hydrocarbon group having 1 to 6 carbon atoms or an oxygen atom, 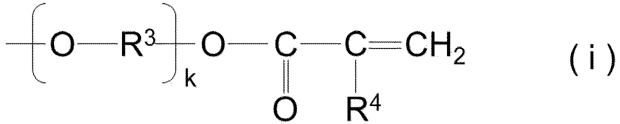 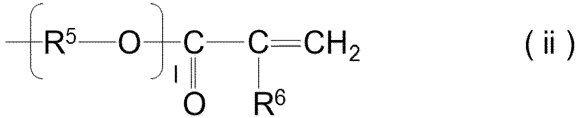 in which in the general formulae (i) and (ii), R³ and R⁵ each independently represent a divalent hydrocarbon group having 1 to 10 carbon atoms, R⁴ and R⁶ each independently represent a hydrogen atom or a methyl group, and k and l each independently represent an integer of 0 to 6.
[2] The dental curable composition according to the item [1], in which R⁴ and R⁶ each represent a methyl group.
[3] The dental curable composition according to the item [1] or [2], in which k and l each are 0 or 1.
[4] The dental curable composition according to any one of the items [1] to [3], in which the component (B) contains the compound (b-II), and X represents an oxygen atom.
[5] The dental curable composition according to any one of the items [1] to [4], in which R² represents a group represented by the general formula (ii).
[6] The dental curable composition according to any one of the items [1] to [5], in which the component (A) contains a (meth)acrylic based compound (a-I) containing a urethane bond.
[7] A dental restorative material containing the dental curable composition according to any one of the items [1] to [6].
[8] A dental cement containing the dental curable composition according to any one of the items [1] to [6].
[9] A dental abutment building material containing the dental curable composition according to any one of the items [1] to [6].

### Advantageous Effects of Invention

The present invention can provide a dental curable composition that has a particularly small polymerization shrinkage stress and is excellent in the mechanical strength of the cured product.

### Description of Embodiments

The present invention will be described in detail with reference to embodiments below.

In the description herein, the upper limit values and the lower limit values of the numeral ranges (such as the contents of the components, the values calculated from the components, and the property values) can be appropriately combined. In the description herein, the values of the symbols in the formulae can also be appropriately combined.

Therefore, in the description herein, the lower limit values and the upper limit values described in a stepwise manner for the numeral ranges can be independently combined. For example, based on the description "preferably 10 to 90, and more preferably 30 to 60" for the same item, the "preferred lower limit value (10)" and the "more preferred upper limit value (60)" can be combined to make a range of "10 to 60".

As for the numeral ranges, for example, based on the description "preferably 10 to 90, and more preferably 30 to 60", only the lower limit value can be defined as "10 or more" or "30 or more" without particularly limiting the upper limit value. Similarly, only the upper limit value can be defined as "90 or less" or "60 or less" without particularly limiting the lower limit value.

Unless otherwise indicated, the description "10 to 90" simply for the numeral range means a range of 10 or more and 90 or less.

As similar to the above, for example, based on the description "preferably 10 or more, and more preferably 30 or more" and the description "preferably 90 or less, and more preferably 60 or less" for the same item, the "preferred lower limit value (10)" and the "more preferred upper limit value (60)" can be combined to make a range of "10 or more and 60 or less". As similar to the above, only the lower limit value can be defined as "10 or more" or "30 or more", and similarly, only the upper limit value can be defined as "90 or less" or "60 or less". This is similarly applied to the case where the upper limit of the numeral range is "less than".

In the description herein, the expression "(meth)acrylic" is used as the concept encompassing both "methacrylic" and "acrylic". This is similarly applied to the analogous expressions including "(meth)acrylate", "(meth)acrylate ester", "(meth)acrylamide", "(meth)acryloyl", "(meth)acryloyloxy", and the like.

In the description herein, the "(meth)acrylic based compound" is used as the concept encompassing both a "(meth)acrylate ester compound" and a "(meth)acrylamide and a derivative thereof.

In the description herein, unless otherwise indicated, the "polymerization shrinkage stress", the "curability", and the "storage stability" mean the "polymerization shrinkage stress", the "curability", and the "storage stability" of the dental curable composition as one embodiment of the present invention. In the description herein, unless otherwise indicated, the "mechanical strength" or "strength" means the "flexural strength" and the "flexural modulus" of a cured product of the dental curable composition as one embodiment of the present invention.

### [Dental Curable Composition]

The dental curable composition as one embodiment of the present invention contains a (meth)acrylic based compound (A) having two or more (meth)acryloyl groups in one molecule (which may be hereinafter referred simply to as a "component (A)"), a mono(meth)acrylate ester compound (B) (which may be hereinafter referred simply to as a "component (B)"), a chemical polymerization initiator (C) (which may be hereinafter referred simply to as a "component (C)"), a polymerization accelerator (D) (which may be hereinafter referred simply to as a "component (D)"), and a filler (E) (which may be hereinafter referred simply to as a "component (E)"), the mono(meth)acrylate ester compound (B) contains at least one kind selected from the group consisting of a mono(meth)acrylate ester compound (b-I) represented by the general formula (I) and a mono(meth)acrylate ester compound (b-II) represented by the general formula (II), and the dental curable composition is separately packaged in a first material containing the chemical polymerization initiator (C) and a second material containing the polymerization accelerator (D).

The polymerization initiator system containing the component (C) and the component (D) is used in the dental curable composition as described later, and in a product form of the dental curable composition, the component (C) and the component (D) are generally separately packaged in separate containers from the standpoint of the storage stability of the dental curable composition. Accordingly, in a product form, the dental curable composition is generally provided in the form including at least the first material containing the component (C) and the second material containing the component (D).

In a preferred product form, the dental curable composition as one embodiment of the present invention is provided as a kit used in the form of two-component system including the first material and the second material, and in a more preferred product form, is provided as a kit used in the form of two-paste system, in which both the two materials are in the form of paste. In the kit used in the form of two-component system, it is preferred that the dental curable composition as one embodiment of the present invention includes the first material containing the component (A), the component (B), and the component (C), and the second material containing the component (A), the component (B), and the component (D). In the case of the use thereof in the form of two-paste system, it is preferred that the pastes are stored in such a manner that the pastes are isolated from each other, and immediately before use, the two pastes are kneaded to perform chemical polymerization, or to perform chemical polymerization and photopolymerization in the case where a photopolymerization initiator is further contained, for curing.

The components contained in the dental curable composition as one embodiment of the present invention will be described below.

### <(Meth)acrylic based Compound (A) having Two or more (Meth)acryloyl Groups in One Molecule>

The (meth)acrylic based compound (A) having two or more (meth)acryloyl groups in one molecule is used for imparting curability to the dental curable composition, and for imparting strength to the cured product of the dental curable composition.

The component (A) is roughly classified into a (meth)acrylic based compound (a-I) containing a urethane bond (which may be hereinafter referred simply to as a "compound (a-I)) and a (meth)acrylic based compound (a-II) containing no urethane bond (which may be hereinafter referred simply to as a "compound (a-II)), and the compound (a-I) is preferably contained from the standpoint of achieving the excellent curability of the dental curable composition and facilitating the suppression of the polymerization shrinkage stress.

The component (A) is preferably a (meth)acrylate ester compound having two or more (meth)acryloyl groups in one molecule, and more preferably a (meth)acrylate ester compound having two or more (meth)acryloyloxy groups in one molecule.

The number of the (meth)acryloyl groups in one molecule is not particularly limited, as far as being 2 or more and exerting the effects of the present invention, and in one embodiment of the present invention, for example, the number thereof may be 2 to 10, and is preferably 2 to 6, more preferably 2 to 4, further preferably 2 to 3, and still further preferably 2.

Similarly, the number of the (meth)acryloyloxy groups in one molecule is not particularly limited, as far as being 2 or more and exerting the effects of the present invention, and in one embodiment of the present invention, for example, the number thereof may be 2 to 10, and is preferably 2 to 6, more preferably 2 to 4, further preferably 2 to 3, and still further preferably 2.

### ((Meth)acrylic based Compound (a-I) containing Urethane Bond)

The compound (a-I) can be easily synthesized, for example, by subjecting a compound containing an isocyanate containing an alkylene skeleton or a phenylene skeleton and a (meth)acrylic based compound having a hydroxy group (-OH) to addition reaction, and can be easily synthesized, for example, by subjecting a polyol having a polymer skeleton, a compound having an isocyanate group (-NCO), and a (meth)acrylic based compound having a hydroxy group (-OH) to addition reaction. Among these, it is preferred that the polymer skeleton is not contained from the standpoint of achieving the excellent strength of the cured product of the dental curable composition.

The compound (a-I) is preferably a (meth)acrylate ester compound containing a urethane bond.

Examples of the compound having an isocyanate group include methylene diisocyanate (abbr.: MDI), hexamethylene diisocyanate (abbr.: HDI), isophorone diisocyanate (abbr.: IPDI), trimethylhexamethylene diisocyanate (abbr.: TMHMDI), tricyclodecane diisocyanate (abbr.: TCDDI), adamantane diisocyanate (abbr.: ADI), tolylene diisocyanate (abbr.: TDI), xylylene diisocyanate (abbr.: XDI), and diphenylmethane diisocyanate (abbr.: DPMDI). One kind of these compounds may be used alone, or two or more kinds thereof may be used in combination. Among these, IPDI, TMHMDI, and TCDDI are preferred, and TMHMDI is more preferred, from the standpoint of achieving the excellent strength of the cured product of the dental curable composition.

Examples of the (meth)acrylic based compound having a hydroxy group include a hydroxy (meth)acrylate compound, such as 2-hydroxyethyl (meth)acrylate, 2-hydroxypropyl (meth)acrylate, 2-hydroxybutyl (meth)acrylate, 6-hydroxyhexyl (meth)acrylate, 10-hydroxydecyl (meth)acrylate, 3-chloro-2-hydroxypropyl (meth)acrylate, 2-hydroxy-3-phenoxypropyl (meth)acrylate, glycerin mono(meth)acrylate, N-hydroxyethyl (meth)acrylamide, N,N-bis(2-hydroxyethyl) (meth)acrylamide, 2-hydroxy-3-acryloyloxypropyl (meth)acrylate, 2,2-bis(4-(3-(meth)acryloyloxy-2-hydroxypropoxy)phenyl)propane, 1,2-bis(3-(meth)acryloyloxy-2-hydroxypropoxy)ethane, pentaerythritol tri(meth)acrylate, and dipentaerythritol tri- or tetra(meth)acrylate. One kind of these compounds may be used alone, or two or more kinds thereof may be used in combination. Among these, 2-hydroxyethyl (meth)acrylate, 2-hydroxypropyl (meth)acrylate, and 2-hydroxy-3-acryloyloxypropyl (meth)acrylate are preferred, and 2-hydroxyethyl (meth)acrylate is more preferred, from the standpoint of achieving the excellent curability of the dental curable composition.

The addition reaction of the compound containing an isocyanate group and the (meth)acrylic based compound having a hydroxy group can be performed according to the ordinary method with no particular limitation.

Examples of the compound (a-I) having no polymer skeleton in the compound (a-I) include 2,2,4-trimethylhexamethylenebis(2-carbamoyloxyethyl) dimethacrylate (abbr.: UDMA), 2,4-tolylenebis(2-carbamoyloxyethyl) dimethacrylate, bishydroxyethyl methacrylate-isophorone diurethane, 2,4-tolylenebis(2-carbamoyloxyethyl) dimethacrylate, N,N'-(2,2,4-trimethylhexamethylene)bis[2-(aminocarboxy)propan-1,3-diol] tetramethacrylate (abbr.: U4TH), hexamethylenebis(2-carbamoyloxy-3-phenoxypropyl) diacrylate, and 2,4-tolylenebis(2-carbamoyloxyethyl) hexaacrylate. One kind of these compounds may be used alone, or two or more kinds thereof may be used in combination. Among these, UDMA and U4TH are preferred, and UDMA is more preferred, from the standpoint of facilitating the strength and the reduction of the polymerization shrinkage stress.

The content of the compound (a-I) in the dental curable composition is preferably 50 parts by mass or more per 100 parts by mass in total of the compound (a-I) and the compound (a-II), is more preferably 75 parts by mass or more, and further preferably 90 parts by mass or more, from the standpoint of facilitating the reduction of the polymerization shrinkage stress, is 100 parts by mass or less, and may be 100 parts by mass. In other words, in one embodiment of the present invention, the content of the compound (a-I) in the dental curable composition is preferably 50 to 100 parts by mass, more preferably 75 to 100 parts by mass, and further preferably 90 to 100 parts by mass, and may be 100 parts by mass, per 100 parts by mass in total of the compound (a-I) and the compound (a-II).

In one embodiment of the dental curable composition, the content of the compound (a-I) in the dental curable composition may be 50 parts by mass or less, may be 25 parts by mass or less, may be 10 parts by mass or less, is 0 part by mass or more, and may be 0 part by mass, per 100 parts by mass in total of the compound (a-I) and the compound (a-II). In other words, in one embodiment of the dental curable composition, the content of the compound (a-I) in the dental curable composition may be 0 to 50 parts by mass, may be 0 to 25 parts by mass, may be 0 to 10 parts by mass, and may be 0 part by mass, per 100 parts by mass in total of the compound (a-I) and the compound (a-II).

### ((Meth)acrylic based Compound (a-II) containing No Urethane Bond)

Examples of the compound (a-II) include an aromatic compound based polymerizable monomer having two (meth)acryloyl groups in one molecule containing no urethane bond; an aliphatic compound based polymerizable monomer having two (meth)acryloyl groups in one molecule containing no urethane bond; and a polymerizable monomer having three or more (meth)acryloyl groups in one molecule containing no urethane bond. One kind of these compounds may be used alone, or two or more kinds thereof may be used in combination.

The compound (a-II) is preferably a (meth)acrylate ester compound containing no urethane bond.

Examples of the aromatic compound based polymerizable monomer having two (meth)acryloyl groups in one molecule containing no urethane bond include 2,2-bis((meth)acryloyloxyphenyl)propane, 2,2-bis(4-(3-(meth)acryloyloxy)-2-hydroxypropoxyphenyl)propane, 2,2-bis(4-(meth)acryloyloxyethoxyphenyl)propane, 2,2-bis(4-(meth)acyrloyloxypolyethoxyphenyl)propane, 2,2-bis(4-(meth)acryloyloxydiethoxyphenyl)propane, 2,2-bis(4-(meth)acryloyloxytetraethoxyphenyl)propane, 2,2-bis(4-(meth)acryloyloxypentaethoxyphenyl)propane, 2,2-bis(4-(meth)acryloyloxydipropoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxyditriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxypropoxyphenyl)propane, 2,2-bis(4-(meth)acryloyloxyisopropoxyphenyl)propane, and 1,4-bis(2-(meth)acryloyloxyethyl) pyromellitate. These compounds may be used alone, or two or more kinds thereof may be used in combination.

Examples of the aliphatic compound based polymerizable monomer having two (meth)acryloyl groups in one molecule containing no urethane bond include glycerol di(meth)acrylate, ethylene glycol di(meth)acrylate, diethylene glycol di(meth)acrylate, triethylene glycol di(meth)acrylate, propylene glycol di(meth)acrylate, butylene glycol di(meth)acrylate, neopentyl glycol di(meth)acrylate, polyethylene glycol di(meth)acrylate, 1,3-butanediol di(meth)acrylate, 1,4-butanediol di(meth)acrylate, 1,6-hexanediol di(meth)acrylate, 2-ethyl-1,6-hexanediol di(meth)acrylate, 1,9-nonanediol di(meth)acrylate, 1,10-decanediol di(meth)acrylate, 1,2-bis(3-methacryloyloxy-2-hydroxypropoxy)ethane, N,N'-ethylenebis(meth)acrylamide, N,N'-propylenebis(meth)acrylamide, N,N'-butylenebis(meth)acrylamide, N,N'-hexamethylenebis(meth)acrylamide, 2-(meth)acryloyloxyethyl(meth)acrylamide, 2-(meth)acryloyloxypropyl(meth)acrylamide, 2-(meth)acryloyloxybutyl(meth)acrylamide, and 2-(meth)acryloyloxyhexyl(meth)acrylamide. One kind of these compounds may be used alone, or two or more kinds thereof may be used in combination.

Examples of the polymerizable monomer having three or more (meth)acryloyl groups in one molecule containing no urethane bond include trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, dipentaerythritol penta(meth)acrylate, and 1,7-diacryloyloxy-2,2,6,6-tetraacryloyloxymethyl-4-oxyheptane. The number of the (meth)acryloyl groups in the polymerizable monomer having three or more (meth)acryloyl groups in one molecule containing no urethane bond is 3 or more, and is not particularly limited, as far as exerting the effects of the present invention, and in one embodiment of the present invention, the number thereof may be 3 to 10, may be 3 to 8, and may be 3 to 6. One kind of these compounds may be used alone, or two or more kinds thereof may be used in combination.

The content of the compound (a-II) in the dental curable composition is preferably 50 parts by mass or less per 100 parts by mass in total of the compound (a-I) and the compound (a-II), is more preferably 25 parts by mass or less, and further preferably 10 parts by mass or less, from the standpoint of facilitating the reduction of the polymerization shrinkage stress, and is 0 part by mass or more. In other words, in one embodiment of the present invention, the content of the compound (a-II) in the dental curable composition is preferably 0 to 50 parts by mass, more preferably 0 to 25 parts by mass, and further preferably 0 to 10 parts by mass, per 100 parts by mass in total of the compound (a-I) and the compound (a-II).

In one embodiment of the dental curable composition, the content of the compound (a-II) in the dental curable composition may be 50 parts by mass or more, may be 75 parts by mass or more, may be 90 parts by mass or more, is 100 parts by mass or less, and may be 100 parts by mass, per 100 parts by mass in total of the compound (a-I) and the compound (a-II). In other words, in one embodiment of the dental curable composition, the content of the compound (a-II) in the dental curable composition may be 50 to 100 parts by mass, may be 75 to 100 parts by mass, may be 90 to 100 parts by mass, and may be 100 parts by mass, per 100 parts by mass in total of the compound (a-I) and the compound (a-II).

The total content of the compound (a-I) and the compound (a-II) in the component (A) is preferably 70 to 100 parts by mass, more preferably 80 to 100 parts by mass, and further preferably 90 to 100 parts by mass, and may be 100 parts by mass, per 100 parts by mass of the component (A), from the standpoint of facilitating the reduction of the polymerization shrinkage stress.

The content of the component (A) in the dental curable composition is preferably 20 to 95 parts by mass per 100 parts by mass in total of the component (A) and the component (B), and is more preferably 40 to 90 parts by mass, and further preferably 60 to 85 parts by mass, from the standpoint of achieving the excellent strength.

### <Mono(meth)acrylate Ester Compound (B)>

The mono(meth)acrylate ester compound (B) is used in the dental curable composition for reducing the viscosity of the dental curable composition, enhancing the strength of the cured product, and imparting the effect of further reducing the polymerization shrinkage stress. The component (B) has a relatively small molecular weight due to the monofunctionality thereof, and contains no polar functional group other than the group represented by R¹ and R², thereby being capable of suppressing the viscosity of the dental curable composition. The glass transition temperature (Tg) of the homopolymer thereof tends to be low due to the monofunctionality thereof, and it is estimated that a soft phase is formed thereby while reducing the crosslinking density of the cured product of the dental curable composition, and thus an effect of relaxing the shrinkage and the strain caused by the polymerization can be exerted. Due to the monofunctionality, on the other hand, there is a concern of the decrease in polymerizability in the form of the dental curable composition, but it is estimated that the radical stabilization effect of the π-electron conjugated system enhances the polymerizability, and thus the strength can be enhanced irrespective of the low glass transition temperature (Tg) of the homopolymer.

It suffices that the component (B) contains at least one kind selected from the group consisting of a mono(meth)acrylate ester compound (b-I) (which may be hereinafter referred simply to as a "compound (b-I)") represented by the general formula (I) and a mono(meth)acrylate ester compound (b-II) (which may be hereinafter referred simply to as a "compound (b-II)") represented by the general formula (II), and a mono(meth)acrylate ester compound containing an aromatic ring is preferred. The compound (b-I) and the compound (b-II) will be described below.

### (Mono(meth)acrylate Ester Compound (b-I) represented by General Formula (I))

The symbols in the general formula (I) showing the compound (b-I) will be described. In the general formula (I), R¹ represents a group represented by the general formula (i) or a group represented by the general formula (ii), and in the general formula (i) or (ii), R⁴ and R⁶ each independently represent a hydrogen atom or a methyl group from the standpoint of achieving the excellent curability of the dental curable composition, and preferably a methyl group from the standpoint of further facilitating the reduction of the polymerization shrinkage stress of the dental curable composition. In the general formula or (i) or (ii), R³ and R⁵ each independently represent a divalent hydrocarbon group having 1 to 10 carbon atoms. The hydrocarbon group preferably has 1 to 6 carbon atoms, more preferably 1 to 4 carbon atoms, and further preferably 1 to 3 carbon atoms, from the standpoint of achieving the low viscosity of the dental curable composition and the excellent curability thereof. Examples of the hydrocarbon group include a linear or branched alkylene group having 1 to 10 carbon atoms, a divalent cycloalkylene group having 3 to 10 carbon atoms, and a phenylene group. In the general formula (i) or (ii), k and l each independently represent an integer of 0 to 6, in which k preferably represents 0 to 4, more preferably 0 to 3, further preferably 0 to 2, and still further preferably 0 or 1, from the standpoint of achieving the low viscosity of the dental curable composition and the excellent curability thereof, and l preferably represents 0 to 4, more preferably 0 to 2, and further preferably 0 or 1. Accordingly, in the general formula (i) or (ii), k and l each still more further preferably represent 0 or 1.

Examples of the compound (b-I) include o-phenylphenol (meth)acrylate, m-phenylphenol (meth)acrylate, p-phenylphenol (meth)acrylate, methoxylated o-phenylphenol (meth)acrylate, methoxylated m-phenylphenol (meth)acrylate, methoxylated p-phenylphenol (meth)acrylate, ethoxylated o-phenylphenol (meth)acrylate, ethoxylated m-phenylphenol (meth)acrylate, ethoxylated p-phenylphenol (meth)acrylate, propoxylated o-phenylphenol (meth)acrylate, propoxylated m-phenylphenol (meth)acrylate, propoxylated p-phenylphenol (meth)acrylate, butoxylated o-phenylphenol (meth)acrylate, butoxylated m-phenylphenol (meth)acrylate, and butoxylated p-phenylphenol (meth)acrylate. One kind of these compounds may be used alone, or two or more kinds thereof may be used in combination. Among these, ethoxylated o-phenylphenol (meth)acrylate, ethoxylated m-phenylphenol (meth)acrylate, ethoxylated p-phenylphenol (meth)acrylate, propoxylated o-phenylphenol (meth)acrylate, propoxylated m-phenylphenol (meth)acrylate, and propoxylated p-phenylphenol (meth)acrylate are preferred, ethoxylated o-phenylphenol (meth)acrylate, ethoxylated m-phenylphenol (meth)acrylate, and ethoxylated p-phenylphenol (meth)acrylate are more preferred, ethoxylated o-phenylphenol (meth)acrylate and ethoxylated m-phenylphenol (meth)acrylate are further preferred, and ethoxylated o-phenylphenol (meth)acrylate is still further preferred, from the standpoint of achieving the curability of the dental curable composition, the effect of reducing the polymerization shrinkage stress thereof, and the excellent strength of the cured product of the dental curable composition, and ethoxylated o-phenylphenol methacrylate is still more further preferred from the standpoint of achieving the further excellent effect of reducing the polymerization shrinkage stress of the dental curable composition.

The content of the compound (b-I) in the dental curable composition is preferably 50 parts by mass or less per 100 parts by mass in total of the compound (b-I) and the compound (b-II), is more preferably 45 parts by mass or less, further preferably 40 parts by mass or less, still further preferably 20 parts by mass or less, still more further preferably 10 parts by mass or less, from the standpoint of facilitating the reduction of the polymerization shrinkage stress, and is 0 part by mass or more. In other words, in one embodiment of the present invention, the content of the compound (b-I) in the dental curable composition is preferably 0 to 50 parts by mass, more preferably 0 to 45 parts by mass, further preferably 0 to 40 parts by mass, still further preferably 0 to 20 parts by mass, and still more further preferably 0 to 10 parts by mass, per 100 parts by mass in total of the compound (b-I) and the compound (b-II).

In one embodiment of the dental curable composition, the content of the compound (b-I) in the dental curable composition may be 20 parts by mass or more, may be 30 parts by mass or more, may be 35 parts by mass or more, may be 60 parts by mass or less, may be 50 parts by mass or less, and may be 45 parts by mass or less, per 100 parts by mass in total of the compound (b-I) and the compound (b-II). As described above, the lower limit values and the upper limit values described in a stepwise manner can be independently combined. For example, in one embodiment of the dental curable composition, the content of the compound (b-I) in the dental curable composition may be 20 to 60 parts by mass, may be 30 to 50 parts by mass, and may be 35 to 45 parts by mass, per 100 parts by mass in total of the compound (b-I) and the compound (b-II).

### (Mono(meth)acrylate Ester Compound (b-II) represented by General Formula (II))

The symbols in the general formula (II) showing the compound (b-II) will be described. In the general formula (II), X represents a divalent hydrocarbon group having 1 to 6 carbon atoms or an oxygen atom, and preferably an oxygen atom from the standpoint of achieving the low viscosity of the dental curable composition and the excellent curability thereof. In the general formula (II), R² represents a group selected from the general formula (i) or (ii), and in the general formula (i) or (ii), R⁴ and R⁶ each independently represent a hydrogen atom or a methyl group from the standpoint of achieving the excellent curability of the dental curable composition, and preferably a methyl group from the standpoint of further facilitating the reduction of the polymerization shrinkage stress of the dental curable composition. In the general formula or (i) or (ii), R³ and R⁵ each independently represent a divalent hydrocarbon group having 1 to 10 carbon atoms. The hydrocarbon group preferably has 1 to 6 carbon atoms, more preferably 1 to 4 carbon atoms, and further preferably 1 to 3 carbon atoms, from the standpoint of achieving the low viscosity of the dental curable resin composition and the excellent curability thereof. Examples of the hydrocarbon group include a linear or branched alkylene group having 1 to 10 carbon atoms, a divalent cycloalkylene group having 3 to 10 carbon atoms, and a phenylene group. In the general formula (i) or (ii), k and l each independently represent an integer of 0 to 6, in which k preferably represents 0 to 4, more preferably 0 to 3, further preferably 0 to 2, and still further preferably 0 or 1, from the standpoint of achieving the low viscosity of the dental curable composition and the excellent curability thereof, and l preferably represents 0 to 4, more preferably 0 to 2, and further preferably 0 or 1. Accordingly, in the general formula (i) or (ii), k and l each still more further preferably represent 0 or 1.

In the general formula (II), R² preferably represents a group selected from the general formula (ii) from the standpoint of the curability.

Examples of the compound (b-II) include o-phenoxybenzyl (meth)acrylate, m-phenoxybenzyl (meth)acrylate, p-phenoxybenzyl (meth)acrylate, 2-(o-phenoxyphenyl)ethyl (meth)acrylate, 2-(m-phenoxyphenyl)ethyl (meth)acrylate, 2-(p-phenoxyphenyl)ethyl (meth)acrylate, 3-(o-phenoxyphenyl)propyl (meth)acrylate, 3-(m-phenoxyphenyl)propyl (meth)acrylate, 3-(p-phenoxyphenyl)propyl (meth)acrylate, 4-(o-phenoxyphenyl)butyl (meth)acrylate, 4-(m-phenoxyphenyl)butyl (meth)acrylate, 4-(p-phenoxyphenyl)butyl (meth)acrylate, 5-(o-phenoxyphenyl)pentyl (meth)acrylate, 5-(m-phenoxyphenyl)pentyl (meth)acrylate, 5-(p-phenoxyphenyl)pentyl (meth)acrylate, 6-(o-phenoxyphenyl)hexyl (meth)acrylate, 6-(m-phenoxyphenyl)hexyl (meth)acrylate, and 6-(p-phenoxyphenyl)hexyl (meth)acrylate. One kind of these compounds may be used alone, or two or more kinds thereof may be used in combination. Among these, o-phenoxybenzyl (meth)acrylate, m-phenoxybenzyl (meth)acrylate, p-phenoxybenzyl (meth)acrylate, 2-(o-phenoxyphenyl)ethyl (meth)acrylate, 2-(m-phenoxyphenyl)ethyl (meth)acrylate, and 2-(p-phenoxyphenyl)ethyl (meth)acrylate are preferred, o-phenoxybenzyl (meth)acrylate, m-phenoxybenzyl (meth)acrylate, and p-phenoxybenzyl (meth)acrylate are more preferred, o-phenoxybenzyl (meth)acrylate and m-phenoxybenzyl (meth)acrylate are further preferred, and m-phenoxybenzyl (meth)acrylate is still further preferred, from the standpoint of achieving the curability of the dental curable composition, the effect of reducing the polymerization shrinkage stress thereof, and the excellent strength of the cured product of the dental curable composition, in which m-phenoxybenzyl methacrylate is still more further preferred from the standpoint of achieving the further excellent effect of reducing the polymerization shrinkage stress of the dental curable composition.

The content of the compound (b-II) in the dental curable composition is preferably 50 parts by mass or more per 100 parts by mass in total of the compound (b-I) and the compound (b-II), is more preferably 55 parts by mass or more, further preferably 60 parts by mass or more, still further preferably 80 parts by mass or more, still more further preferably 90 parts by mass or more, from the standpoint of facilitating the reduction of the polymerization shrinkage stress, is 100 parts by mass or less, and may be 100 parts by mass. In other words, in one embodiment of the present invention, the content of the compound (b-II) in the dental curable composition is preferably 50 to 100 parts by mass, more preferably 55 to 100 parts by mass, further preferably 60 to 100 parts by mass, still further preferably 80 to 100 parts by mass, and still more further preferably 90 to 100 parts by mass, and may be 100 parts by mass, per 100 parts by mass in total of the compound (b-I) and the compound (b-II).

In one embodiment of the dental curable composition, the content of the compound (b-II) in the dental curable composition may be 40 parts by mass or more, may be 50 parts by mass or more, may be 55 parts by mass or more, may be 80 parts by mass or less, may be 70 parts by mass or less, and may be 65 parts by mass or less, per 100 parts by mass in total of the compound (b-I) and the compound (b-II). As described above, the lower limit values and the upper limit values described in a stepwise manner can be independently combined. For example, in one embodiment of the dental curable composition, the content of the compound (b-II) in the dental curable composition may be 40 to 80 parts by mass, may be 50 to 70 parts by mass, and may be 55 to 65 parts by mass, per 100 parts by mass in total of the compound (b-I) and the compound (b-II).

The total content of the compound (b-I) and the compound (b-II) in the component (B) is preferably 70 to 100 parts by mass, more preferably 80 to 100 parts by mass, and further preferably 90 to 100 parts by mass, and may be 100 parts by mass, per 100 parts by mass of the component (B), from the standpoint of facilitating the reduction of the polymerization shrinkage stress.

The content of the component (B) in the dental curable composition is preferably 5 to 80 parts by mass per 100 parts by mass in total of the component (A) and the component (B), and is more preferably 10 to 60 parts by mass, and further preferably 15 to 40 parts by mass, from the standpoint of achieving the further excellent strength of the cured product and the further excellent effect of reducing the polymerization shrinkage stress.

The total amount of the component (A) and the component (B) in the dental curable composition is preferably 10 to 60% by mass, more preferably 15 to 55% by mass, further preferably 20 to 50% by mass, and still further preferably 25 to 45% by mass, based on 100% by mass of the dental curable composition, from the standpoint of further facilitating the effects of the present invention.

The dental curable composition may contain a mono(meth)acrylate ester compound other than the component (B) for the purposes of regulating the adhesiveness and the viscosity, and the like. Examples of the mono(meth)acrylate ester compound other than the component (B) include 2-hydroxyethyl (meth)acrylate, 2-hydroxypropyl (meth)acrylate, 3-hydroxypropyl (meth)acrylate, 4-hydroxybutyl (meth)acrylate, 6-hydroxyhexyl (meth)acrylate, 10-hydroxydecyl (meth)acrylate, propylene glycol mono(meth)acrylate, glycerol mono(meth)acrylate, erythritol mono(meth)acrylate, N-methylol(meth)acrylamide, N-hydroxyethyl(meth)acrylamide, N,N-(dihydroxyethyl)(meth)acrylamide, methyl (meth)acrylate, ethyl (meth)acrylate, propyl (meth)acrylate, isopropyl (meth)acrylate, n-butyl (meth)acrylate, sec-butyl (meth)acrylate, t-butyl (meth)acrylate, isobutyl (meth)acrylate, n-hexyl (meth)acrylate, cyclohexyl (meth)acrylate, 2-ethylhexyl (meth)acrylate, lauryl (meth)acrylate, cetyl (meth)acrylate, stearyl (meth)acrylate, isobornyl (meth)acrylate, benzyl (meth)acrylate, phenyl (meth)acrylate, 2-phenoxyethyl (meth)acrylate, 2,3-dibromopropyl (meth)acrylate, 3-(meth)acryloyloxypropyltrimethoxysilane, 11-(meth)acryloyloxyundecyltrimethoxysilane, (meth)acrylamide, 2-(meth)acryloyloxyethyl dihydrogen phosphate, 3-(meth)acryloyloxypropyl dihydrogen phosphate, 4-(meth)acryloyloxybutyl dihydrogen phosphate, 5-(meth)acryloyloxypentyl dihydrogen phosphate, 6-(meth)acryloyloxyhexyl dihydrogen phosphate, 7-(meth)acryloyloxyheptyl dihydrogen phosphate, 8-(meth)acryloyloxyoctyl dihydrogen phosphate, 9-(meth)acryloyloxynonyl dihydrogen phosphate, 10-(meth)acryloyloxy decyl dihydrogen phosphate, 11-(meth)acryloyloxyundecyl dihydrogen phosphate, 12-(meth)acryloyloxydodecyl dihydrogen phosphate, 16-(meth)acryloyloxyhexadecyl dihydrogen phosphate, 20-(meth)acryloyloxyicosyl dihydrogen phosphate, bis(2-(meth)acryloyloxyethyl) hydrogen phosphate, 2-(meth)acryloyloxyethylphenyl hydrogen phosphate, and 2-(meth)acryloyloxyethyl-2-bromoethyl hydrogen phosphate.

The polymerizable monomer contained in the dental curable composition may be constituted substantially only by the component (A) and the component (B). The fact that the polymerizable monomer is constituted substantially only by the component (A) and the component (B) means that the content of the polymerizable monomer other than the component (A) and the component (B) is less than 10.0% by mass, preferably less than 5.0% by mass, more preferably less than 1.0% by mass, further preferably less than 0.1% by mass, and still further preferably less than 0.01% by mass, based on the total amount of 100% by mass of the polymerizable monomer contained in the dental curable composition. In other words, in one embodiment of the present invention, the fact that the polymerizable monomer is constituted substantially only by the component (A) and the component (B) means that the content of the polymerizable monomer other than the component (A) and the component (B) is 0% by mass or more and less than 10.0% by mass, preferably 0% by mass or more and less than 5.0% by mass, more preferably 0% by mass or more and less than 1.0% by mass, further preferably 0% by mass or more and less than 0.1% by mass, and still further preferably 0% by mass or more and less than 0.01% by mass, based on the total amount of 100% by mass of the polymerizable monomer contained in the dental curable composition.

### <Chemical Polymerization Initiator (C)>

The chemical polymerization initiator (C) used may be selected from chemical polymerization initiators that have been used in the general industries, and among these, a chemical polymerization initiator that has been applied to the dental use is preferably used.

The component (C) used is preferably an organic peroxide. The organic peroxide used as the component (C) is not particularly limited, and known compounds can be used. Representative examples of the organic peroxide include a ketone peroxide, a hydroperoxide, a diacyl peroxide, a dialkyl peroxide, a peroxyketal, a peroxyester, and a peroxydicarbonate. One kind of these compounds may be used alone, or two or more kinds thereof may be used in combination.

Examples of the ketone peroxide include methyl ethyl ketone peroxide, methyl isobutyl ketone peroxide, methylcyclohexanone peroxide, and cyclohexanone peroxide.

Examples of the hydroperoxide include 2,5-dimethylhexane-2,5-dihydroperoxide, diisopropylbenzene hydroperoxide, cumene hydroperoxide, t-butyl hydroperoxide, and 1,1,3,3-tetramethylbutyl hydroperoxide.

Examples of the diacyl peroxide include acetyl peroxide, isobutyryl peroxide, benzoyl peroxide, decanoyl peroxide, 3,5,5-trimethylhexanoyl peroxide, 2,4-dichlorobenzoyl peroxide, and lauroyl peroxide.

Examples of the dialkyl peroxide include di-t-butyl peroxide, dicumyl peroxide, t-butyl cumyl peroxide, 2,5-dimethyl-2,5-di(t-butylperoxy)hexane, 1,3-bis(t-butylperoxyisopropyl)benzene, and 2,5-dimethyl-2,5-di(t-butylperoxy)-3-hexyne.

Examples of the peroxyketal include 1,1-bis(t-butylperoxy)-3,3,5-trimethylcyclohexane, 1,1-bis(t-butylperoxy)cyclohexane, 2,2-bis(t-butylperoxy)butane, 2,2-bis(t-butylperoxy)octane, and n-butyl 4,4-bis(t-butylperoxy)valerate.

Examples of the peroxyester include α-cumyl peroxyneodecanoate, t-butyl peroxyneodecanoate, t-butyl peroxypivalate, 2,2,4-trimethylpentyl peroxy-2-ethylhexanoate, t-amyl peroxy-2-ethylhexanoate, t-butyl peroxy-2-ethylhexanoate, di-t-butyl peroxyisophthalate, di-t-butyl peroxyhexahydrophthalate, t-butyl peroxy-3,3,5-trimethylhexanoate, t-butyl peroxyacetate, t-butyl peroxybenzoate, and t-butyl peroxymaleate.

Examples of the peroxydicarbonate include di-3-methoxy peroxydicarbonate, di-2-ethylhexyl peroxydicarbonate, bis(4-t-butylcyclohexyl) peroxydicarbonate, diisopropyl peroxydicarbonate, di-n-propyl peroxydicarbonate, di-2-ethoxyethyl peroxydicarbonate, and diallyl peroxydicarbonate.

Among the organic peroxides, a hydroperoxide and a diacyl peroxide are preferably used, and among these, a hydroperoxide is more preferably used, from the standpoint of the overall balance of the safety, the storage stability, and the radical generating capability. As more specific examples, benzoyl peroxide and 1,1,3,3-tetramethylbutyl hydroperoxide are preferably used, and 1,1,3,3-tetramethylbutyl hydroperoxide is more preferably used, from the standpoint of the overall balance of the safety, the storage stability, and the radical generating capability.

The content of the component (C) is not particularly limited, and the content of the component (C) is preferably 0.01 to 15 parts by mass per 100 parts by mass in total of the component (A) and the component (B), from the standpoint of the curability of the dental curable composition, and the like. In the case where the content of the component (C) is 0.01 part by mass or more, the polymerization can be allowed to proceed sufficiently to prevent stickiness. From this standpoint, the content of the component (C) is more preferably 0.1 part by mass or more, and further preferably 0.5 part by mass or more, per 100 parts by mass in total of the component (A) and the component (B). In the case where the content of the component (C) is 15 parts by mass or less, the deposition or separation of the component (C) from the dental curable composition can be prevented. From this standpoint, the content of the component (C) is more preferably 10 parts by mass or less, and more preferably 5 parts by mass or less, per 100 parts by mass in total of the component (A) and the component (B). As described above, the lower limit values and the upper limit values described in a stepwise manner can be independently combined. For example, in one embodiment of the dental curable composition, the content of the component (C) is preferably 0.01 to 15 parts by mass, more preferably 0.1 to 10 parts by mass, and further preferably 0.5 to 5 parts by mass, per 100 parts by mass in total of the component (A) and the component (B).

### <Polymerization Accelerator (D)>

Examples of the polymerization accelerator (D) include an amine compound, a thiourea compound, sulfinic acid and a salt thereof, a sulfite salt, a bisulfite salt, an aldehyde compound, an organic phosphorus compound, a borate compound, barbituric acid and a derivative thereof, a triazine compound, a vanadium compound, a copper compound, a tin compound, a cobalt compound, a halogen compound, and a thiol compound. One kind of these compounds may be used alone, or two or more kinds thereof may be used in combination.

The amine compound is classified into an aliphatic amine and an aromatic amine.

Examples of the aliphatic amine include a primary aliphatic amine, such as n-butylamine, n-hexylamine, and n-octylamine; a secondary aliphatic amine, such as diisopropylamine, dibutylamine, and N-methylethanolamine; and a tertiary aliphatic amine, such as N-methyldiethanolamine, N-ethyldiethanolamine, N-n-butyldiethanolamine, N-lauryldiethanolamine, 2-(dimethylamino)ethyl methacrylate, N-methyldiethanolamine dimethacrylate, N-ethyldiethanolamine dimethacrylate, triethanolamine monomethacrylate, triethanolamine dimethacrylate, triethanolamine trimethacrylate, triethanolamine, trimethylamine, triethylamine, and tributylamine.

Examples of the aromatic amine include N,N-bis(2-hydroxyethyl)-3, 5-dimethylaniline, N,N-bis(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)4-t-butylaniline, N,N-bis(2-hydroxyethyl)-3,5-diisopropylaniline, N,N-bis(2-hydroxyethyl)-3,5-di-t-butylaniline, N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, N,N-dimethyl-3,5-di-t-butylaniline, ethyl 4-(N,N-dimethylamino)benzoate, methyl 4-(N,N-dimethylamino)benzoate, n-butoxyethyl 4-(N,N-dimethylamino)benzoate, 2-(methacryloyloxy)ethyl 4-(N,N-dimethylamino)benzoate, 4-(N,N-dimethylamino)benzophenone, and butyl 4-(N,N-dimethylamino)benzoate.

Among these, at least one kind selected from the group consisting of N,N-di(2-hydroxyethyl)-p-toluidine, ethyl 4-N,N-dimethylaminobenzoate, n-butoxyethyl N,N-dimethylaminobenzoate, and 4-N,N-dimethylaminobenzophenone is preferably used from the standpoint of imparting the excellent curability to the dental curable composition.

Examples of the thiourea compound include 1-(2-pyridyl)-2-thiourea, thiourea, methylthiourea, ethylthiourea, N,N'-dimethylthiourea, N,N'-diethylthiourea, N,N'-di-n-propylthiourea, N,N'-dicyclohexylthiourea, trimethylthiourea, triethylthiourea, tri-n-propylthiourea, tricyclohexylthiourea, tetramethylthiourea, tetraethylthiourea, tetra-n-propylthiourea, tetracyclohexylthiourea, ethylenethiourea, methylethylenethiourea, and dimethylethylenethiourea.

Among these, at least one kind selected from the group consisting of 1-(2-pyridyl)-2-thiourea and 4,4-dimethylethylenethiourea is preferably used from the standpoint of the curability of the dental curable composition.

Examples of the sulfinic acid and a salt thereof include p-toluenesulfinic acid, sodium p-toluenesulfinate, potassium p-toluenesulfinate, lithium p-toluenesulfinate, calcium p-toluenesulfinate, benzenesulfinic acid, sodium benzenesulfinate, potassium benzenesulfinate, lithium benzenesulfinate, calcium benzenesulfinate, 2,4,6-trimethylbenzenesulfinic acid, sodium 2,4,6-trimethylbenzenesulfinate, potassium 2,4,6-trimethylbenzenesulfinate, lithium 2,4,6-trimethylbenzenesulfinate, calcium 2,4,6-trimethylbenzenesulfinate, 2,4,6-triethylbenzenesulfinic acid, sodium 2,4,6-triethylbenzenesulfinate, potassium 2,4,6-triethylbenzenesulfinate, lithium 2,4,6-triethylbenzenesulfinate, calcium 2,4,6-triethylbenzenesulfinate, 2,4,6-triisopropylbenzenesulfinic acid, sodium 2,4,6-triisopropylbenzenesulfinate, potassium 2,4,6-triisopropylbenzenesulfinate, lithium 2,4,6-triisopropylbenzenesulfinate, and calcium 2,4,6-triisopropylbenzenesulfinate.

Examples of the sulfite salt and the bisulfite salt include sodium sulfite, potassium sulfite, calcium sulfite, ammonium sulfite, sodium bisulfite, and potassium bisulfite.

Examples of the aldehyde compound include terephthalaldehyde and a benzaldehyde derivative. Examples of the benzaldehyde derivative include dimethylaminobenzaldehyde, p-methyloxybenzaldehyde, p-ethyloxybenzaldehyde, and p-n-octyloxybenzaldehyde.

Examples of the organic phosphorus compound include triphenylphosphine, 2-methyltriphenylphosphine, 4-methyltriphenylphosphine, 2-methoxytriphenylphosphine, 4-methoxytriphenylphosphine, tri-n-butylphosphine, triisobutylphosphine, and tri-t-butylphosphine.

The borate compound is preferably an arylborate compound. As specific examples of the arylborate compound that can be preferably used, examples of the borate compound having one aryl group in one molecule include a trialkylphenylboron, a trialkyl(p-chlorophenyl)boron, a trialkyl(p-fluorophenyl)boron, a trialkyl(3,5-bis(trifluoromethyl)phenyl)boron, a trialkyl(3,5-bis(1,1,1,3,3,3-hexafluoro-2-methoxy-2-propyl)phenyl)boron, a trialkyl(p-nitrophenyl)boron, a trialkyl(m-nitrophenyl)boron, a trialkyl(p-butylphenyl)boron, a trialkyl(m-butylphenyl)boron, a trialkyl(p-butyloxyphenyl)boron, a trialkyl(m-butyloxyphenyl)boron, a trialkyl(p-octyloxyphenyl)boron, and a trialkyl(m-octyloxyphenyl)boron (in which the alkyl group is at least one kind selected from the group consisting of a n-butyl group, a n-octyl group, a n-dodecyl group, and the like), and salts thereof (such as a sodium salt, a lithium salt, a potassium salt, a magnesium salt, a tetrabutylammonium salt, a tetramethylammonium salt, a tetraethylammonium salt, a methylpyridinium salt, an ethylpyridinium salt, a butylpyridinium salt, a methylquinolinium salt, an ethylquinolinium salt, and a butylquinolinium salt).

Examples of the borate compound having two aryl groups in one molecule include a dialkyldiphenylboron, a dialkyldi(p-chlorophenyl)boron, a dialkyldi(p-fluorophenyl)boron, a dialkyldi(3,5-bis(trifluoromethyl)phenyl)boron, a dialkyldi(3,5-bis(1,1,1,3,3,3-hexafluoro-2-methoxy-2-propyl)phenyl)boron, a dialkyldi(p-nitrophenyl)boron, a dialkyldi(m-nitrophenyl)boron, a dialkyldi(p-butylphenyl)boron, a dialkyldi(m-butylphenyl)boron, a dialkyldi(p-butyloxyphenyl)boron, a dialkyldi(m-butyloxyphenyl)boron, a dialkyldi(p-octyloxyphenyl)boron, and a dialkyldi(m-octyloxyphenyl)boron (in which the alkyl group is at least one kind selected from the group consisting of a n-butyl group, a n-octyl group, a n-dodecyl group, and the like), and salts thereof (such as a sodium salt, a lithium salt, a potassium salt, a magnesium salt, a tetrabutylammonium salt, a tetramethylammonium salt, a tetraethylammonium salt, a methylpyridinium salt, an ethylpyridinium salt, a butylpyridinium salt, a methylquinolinium salt, an ethylquinolinium salt, and a butylquinolinium salt).

Examples of the borate compound having three aryl groups in one molecule include a monoalkyltriphenylboron, a monoalkyltri(p-chlorophenyl)boron, a monoalkyltri(p-fluorophenyl)boron, a monoalkyltri(3,5-bis(trifluoromethyl)phenyl)boron, a monoalkyltri(3,5-bis(1,1,1,3,3,3-hexafluoro-2-methoxy-2-propyl)phenyl)boron, a monoalkyltri(p-nitrophenyl)boron, a monoalkyltri(m-nitrophenyl)boron, a monoalkyltri(p-butylphenyl)boron, a monoalkyltri(m-butylphenyl)boron, a monoalkyltri(p-butyloxyphenyl)boron, a monoalkyltri(m-butyloxyphenyl)boron, a monoalkyltri(p-octyloxyphenyl)boron, and a monoalkyltri(m-octyloxyphenyl)boron (in which the alkyl group is one kind selected from a n-butyl group, a n-octyl group, a n-dodecyl group, and the like), and salts thereof (such as a sodium salt, a lithium salt, a potassium salt, a magnesium salt, a tetrabutylammonium salt, a tetramethylammonium salt, a tetraethylammonium salt, a methylpyridinium salt, an ethylpyridinium salt, a butylpyridinium salt, a methylquinolinium salt, an ethylquinolinium salt, and a butylquinolinium salt).

Examples of the borate compound having four aryl groups in one molecule include tetraphenylboron, tetrakis(p-chlorophenyl)boron, tetrakis(p-fluorophenyl)boron, tetrakis(3,5-bis(trifluoromethyl)phenyl)boron, tetrakis(3,5-bis(1,1,1,3,3,3-hexafluoro-2-methoxy-2-propyl)phenyl)boron, tetrakis(p-nitrophenyl)boron, tetrakis(m-nitrophenyl)boron, tetrakis(p-butylphenyl)boron, tetrakis(m-butylphenyl)boron, tetrakis(p-butyloxyphenyl)boron, tetrakis(m-butyloxyphenyl)boron, tetrakis(p-octyloxyphenyl)boron, tetrakis(m-octyloxyphenyl)boron, (p-fluorophenyl)triphenylboron, (3,5-bis(trifluoromethyl)phenyl)triphenylboron, (p-nitrophenyl)triphenylboron, (m-butyloxyphenyl)triphenylboron, (p-butyloxyphenyl)triphenylboron, (m-octyloxyphenyl)triphenylboron, (p-octyloxyphenyl)triphenylboron, and salts thereof (such as a sodium salt, a lithium salt, a potassium salt, a magnesium salt, a tetrabutylammonium salt, a tetramethylammonium salt, a tetraethylammonium salt, a methylpyridinium salt, an ethylpyridinium salt, a butylpyridinium salt, a methylquinolinium salt, an ethylquinolinium salt, and a butylquinolinium salt).

Among these arylborate compounds, a borate compound having three or four aryl groups in one molecule is more preferably used from the standpoint of the storage stability. One kind of the borate compound may be used alone, or two or more kinds thereof may be used as a mixture.

Examples of the barbituric acid compound and a derivative thereof include barbituric acid, 1,3-dimethylbarbituric acid, 1,3-diphenylbarbituric acid, 1,5-dimethylbarbituric acid, 5-butylbarbituric acid, 5-ethylbarbituric acid, 5-isopropylbarbituric acid, 5-cyclohexylbarbituric acid, 1,3,5-trimethylbarbituric acid, 1,3-dimethyl-5-ethylbarbituric acid, 1,3-dimethyl-5-n-butylbarbituric acid, 1,3-dimethyl-5-isobutylbarbituric acid, 1,3-dimethyl-5-cyclopentylbarbituric acid, 1,3-dimethyl-5-cyclohexylbarbituric acid, 1,3-dimethyl-5-phenylbarbituric acid, 1-cyclohexyl-1-ethylbarbituric acid, 1-benzyl-5-phenylbarbituric acid, 5-methylbarbituric acid, 5-propylbarbituric acid, 1,5-diethylbarbituric acid, 1-ethyl-5-methylbarbituric acid, 1-ethyl-5-isobutylbarbituric acid, 1,3-diethyl-5-butylbarbituric acid, 1-cyclohexyl-5-methylbarbituric acid, 1-cyclohexyl-5-ethylbarbituric acid, 1-cyclohexyl-5-octylbarbituric acid, 1-cyclohexyl-5-hexylbarbituric acid, 5-butyl-1-cyclohexylbarbituric acid, 1-benzyl-5-phenylbarbituric acid, a thiobarbituric acid compound, and salts thereof (in which an alkali metal salt and an alkaline earth metal salt are preferred). Examples of the salt of the barbituric acid compound include sodium 5-butylbarbiturate, sodium 1,3,5-trimethylbarbiturate, and sodium 1-cyclohexyl-5-ethylbarbiturate.

Examples of the triazine compound include 2,4,6-tris(trichloromethyl)-s-triazine, 2,4,6-tris(tribromomethyl)-s-triazine, 2-methyl-4,6-bis(trichloromethyl)-s-triazine, 2-methyl-4,6-bis(tribromomethyl)-s-triazine, 2-phenyl-4,6-bis(trichloromethyl)-s-triazine, 2-(p-methoxyphenyl)-4,6-bis(trichloromethyl)-s-triazine, 2-(p-methylthiophenyl)-4,6-bis(trichloromethyl)-s-triazine, 2-(p-chlorophenyl)-4,6-bis(trichloromethyl)-s-triazine, 2-(2,4-dichlorophenyl)-4,6-bis(trichloromethyl)-s-triazine, 2-(p-bromophenyl)-4,6-bis(trichloromethyl)-s-triazine, 2-(p-tolyl)-4,6-bis(trichloromethyl)-s-triazine, 2-n-propyl-4,6-bis(trichloromethyl)-s-triazine, 2-(α,α,(3-trichloroethyl)-4,6-bis(trichloromethyl)-s-triazine, 2-styryl-4,6-bis(trichloromethyl)-s-triazine, 2-(2-(p-methoxyphenyl)ethenyl)-4,6-bis(trichloromethyl)-s-triazine, 2-(2-(o-methoxyphenyl)ethenyl)-4,6-bis(trichloromethyl)-s-triazine, 2-(2-(p-butoxyphenyl)ethenyl)-4,6-bis(trichloromethyl)-s-triazine, 2-(2-(3,4-dimethoxyphenyl)ethenyl)-4,6-bis(trichloromethyl)-s-triazine, 2-(2-(3,4,5-trimethoxyphenyl)ethenyl)-4,6-bis(trichloromethyl)-s-triazine, 2-(1-naphthyl)-4,6-bis(trichloromethyl)-s-triazine, 2-(4-biphenylyl)-4,6-bis(trichloromethyl)-s-triazine, 2-(2-(N,N-bis(2-hydroxyethyl)amino)ethoxy)-4,6-bis(trichloromethyl)-s-triazine, 2-(2-(N-hydroxyethyl-N-ethylamino)ethoxy)-4,6-bis(trichloromethyl)-s-triazine, 2-(2-(N-hydroxyethyl-N-methylamino)ethoxy)-4,6-bis(trichloromethyl)-s-triazine, and 2-(2-(N,N-diallylamino)ethoxy)-4,6-bis(trichloromethyl)-s-triazine.

The vanadium compound is preferably a tetravalent and/or pentavalent vanadium compound. Examples of the tetravalent and/or pentavalent vanadium compound include divanadium(IV) tetroxide, vanadyl(IV) acetylacetonate, vanadyl(IV) oxalate, vanadyl(IV) sulfate, vanadium(IV) oxobis(1-phenyl-1,3-butanedionate), oxovanadium(IV) bis(maltolate), vanadium(V) pentoxide, sodium metavanadate(V), and ammonium metavanadate(V). Among these, vanadyl(IV) acetylacetonate is preferably used from the standpoint of the curability of the dental curable composition.

Examples of the copper compound include copper acetylacetonate, cupric acetate, copper oleate, cupric chloride, and cupric bromide. Among these, at least one kind selected from the group consisting of copper acetylacetonate and cupric acetate is preferably used from the standpoint of the curability of the dental curable composition.

Examples of the tin compound include di-n-butyltin dimaleate, di-n-octyltin dimaleate, di-n-octyltin dilaurate, and di-n-butyltin dilaurate. Among these, at least one kind selected from the group consisting of di-n-octyltin dilaurate and di-n-butyltin dilaurate is preferably used.

Examples of the cobalt compound include cobalt acetylacetonate, cobalt acetate, cobalt oleate, cobalt chloride, and cobalt bromide.

Examples of the halogen compound that is preferably used include dilauryldimethylammonium chloride, lauryldimethylbenzylammonium chloride, benzyltrimethylammonium chloride, tetramethylammonium chloride, benzyldimethylcetylammonium chloride, and dilauryldimethylammonium bromide.

Examples of the thiol compound include 3-mercaptopropyltrimethoxysilane, 2-mercaptobenzoxazole, decanethiol, and thiobenzoic acid.

Among the polymerization accelerators (D), at least one kind selected from the group consisting of an amine compound, a thiourea compound, a vanadium compound, and a copper compound is more preferably used, and among these, at least one kind selected from the group consisting of 1-(2-pyridyl)-2-thiourea, N,N-di(2-hydroxyethyl)-p-toluidine, ethyl 4-(N,N-dimethylamino)benzoate, 4,4-dimethylethylenethiourea, vanadyl(IV) acetylacetonate, copper acetylacetonate, and cupric acetate is further preferably used as the component (D).

The content of the component (D) is not particularly limited, and the content of the component (D) is preferably 0.001 to 30 parts by mass per 100 parts by mass in total of the component (A) and the component (B) from the standpoint of the curability of the dental curable composition, and the like. In the case where the content of the component (D) is 0.001 part by mass or more, the polymerization can be allowed to proceed sufficiently to prevent stickiness. From this standpoint, the content of the component (D) is more preferably 0.05 part by mass or more, and further preferably 0.1 part by mass or more, per 100 parts by mass in total of the component (A) and the component (B). In the case where the content of the component (D) is 30 parts by mass or less, the deposition of the component (D) from the dental curable composition can be prevented. From this standpoint, the content of the component (D) is more preferably 20 parts by mass or less, more preferably 10 parts by mass or less, and further preferably 5.0 parts by mass or less, per 100 parts by mass in total of the component (A) and the component (B). As described above, the lower limit values and the upper limit values described in a stepwise manner can be independently combined. For example, in one embodiment of the present invention, the content of the component (D) in the dental curable composition is preferably 0.001 to 30 parts by mass, more preferably 0.05 to 20 parts by mass, further preferably 0.1 to 10 parts by mass, and still further preferably 0.1 to 5.0 parts by mass, per 100 parts by mass in total of the component (A) and the component (B).

In the present invention, the component (C) and the component (D) are used in combination as a redox polymerization initiator, and in this case, as described above, in a product form of the dental curable composition, the component (C) and the component (D) are generally separately packaged in separate containers from the standpoint of the storage stability of the dental curable composition.

### <Filler (E)>

The dental curable composition further contains a filler (E) from the standpoint of regulating the paste property of the dental curable composition before curing, imparting X-ray radiopacity to the cured product, imparting ion sustained releasability thereto, and the like. Examples of the filler that can be used as the component (E) include an organic filler, an inorganic filler, and an organic-inorganic composite filler.

Examples of the material of the organic filler include polymethyl methacrylate, polyethyl methacrylate, a methyl methacrylate-ethyl methacrylate copolymer, crosslinked polymethyl methacrylate, crosslinked polyethyl methacrylate, polyester, polyamide, polycarbonate, polyphenylene ether, polyoxymethylene, polyvinyl chloride, polystyrene, polyethylene, polypropylene, chloroprene rubber, nitrile rubber, an ethylene-vinyl acetate copolymer, a styrene-butadiene copolymer, an acrylonitrile-styrene copolymer, and an acrylonitrile-styrene-butadiene copolymer. One kind of these materials may be used alone, or two or more kinds thereof may be used as a mixture. The shape of the organic filler is not particularly limited, and can be appropriately selected from an irregularly shaped filler, a spherical filler, and the like, and the particle diameter of the filler can be appropriately selected in use.

Examples of the material of the inorganic filler include quartz, silica, alumina, silica-titania, silica-titania-barium oxide, silica-zirconia, silica-alumina, lanthanum glass, borosilicate glass, soda glass, barium glass, strontium glass, glass ceramics, aluminosilicate glass, barium boroaluminosilicate glass, strontium boroaluminosilicate glass, fluoroaluminosilicate glass, fluoroborosilicate glass, calcium fluoroaluminosilicate glass, strontium fluoroaluminosilicate glass, barium fluoroaluminosilicate glass, strontium fluoroaluminosilicate glass, zinc strontium fluoroaluminosilicate glass, strontium calcium fluoroaluminosilicate glass, strontium fluoroboroaluminosilicate glass, zinc strontium fluoroboroaluminosilicate glass, calcium oxide, calcium silicate, calcium phosphate, sodium fluoride, zinc fluoride, and strontium fluoride. One kind of these materials may be used alone, or two or more kinds thereof may be used as a mixture. The shape of the inorganic filler is not particularly limited, and can be appropriately selected from an irregularly shaped filler, a spherical filler, and the like, and the particle diameter of the filler can be appropriately selected in use.

The inorganic filler may be subjected to a surface treatment with a known surface treating agent, such as a silane coupling agent, in advance depending on necessity for regulating the miscibility thereof with the component (A) and the component (B). Examples of the surface treating agent include vinyltrimethoxysilane, vinyltriethoxysilane, vinyltrichlorosilane, vinyltri(β-methoxyethoxy)silane, 3-methacryloyloxypropyltrimethoxysilane, 11-methacryloyloxyundecyltrimethoxysilane, 3-glycidoxypropyltrimethoxysilane, 3-mercaptopropyltrimethoxysilane, and 3-aminopropyltriethoxysilane.

The method of the surface treatment used may be any known method with no particular limitation, and examples thereof include a method of spraying the surface treating agent on the inorganic filler under vigorous agitation, a method of dispersing or dissolving the inorganic filler and the surface treating agent in a suitable solvent, from which the solvent is then removed, and a method of converting the alkoxy group of the surface treating agent to a silanol group through hydrolysis in an aqueous solution, and attaching the surface treating agent to the surface of the inorganic filler in the aqueous solution, from which water is then removed. In all the methods, the surface treatment can be performed by completing the reaction between the surface of the inorganic filler and the surface treating agent by heating generally to a range of 50 to 150°C.

The organic-inorganic composite filler can be obtained in such a manner that a monomer compound is added to the inorganic filler in advance, so as to make a paste, which is then polymerized and pulverized. Examples of the organic-inorganic composite filler used include a TMPT filler (which is obtained in such a manner that trimethylolpropane methacrylate and a silica filler are mixed, polymerized, and then pulverized). The shape of the organic-inorganic composite filler is not particularly limited, and can be appropriately selected from an irregularly shaped filler, a spherical filler, and the like, and the particle diameter of the filler can be appropriately selected in use.

The average primary particle diameter of the component (E) is preferably 0.001 to 10 µm, more preferably 0.005 to 5.0 µm, further preferably 0.01 to 4.0 µm, and still further preferably 0.04 to 3.0 µm, from the standpoint of the handleability of the dental curable composition, the mechanical strength and the transparency of the cured product thereof, and the like. In the description herein, the average primary particle diameter of the component (E) can be obtained through observation with an optical microscope or an electron microscope. Specifically, observation with an optical microscope is convenient for measuring the particle diameter of 100 nm or more, and observation with an electron microscope is convenient for measuring the particle diameter of less than 100 nm.

The content of the component (E) that has an average primary particle diameter of 0.01 µm or more is preferably 80% by mass or more, more preferably 90% by mass or more, further preferably 95% by mass or more, and still further preferably 98% by mass or more, based on the total amount of 100% by mass of the component (E), from the standpoint of further enhancing the mechanical strength of the cured product of the dental curable composition. In other words, in one embodiment of the present invention, the content of the component (E) that has an average primary particle diameter of 0.01 µm or more is preferably 80 to 100% by mass, more preferably 90 to 100% by mass, further preferably 95 to 100% by mass, and still further preferably 98 to 100% by mass, based on the total amount of 100% by mass of the component (E).

It is further preferred that the dental curable composition contains both the component (E) that has an average primary particle diameter of 0.01 µm or more and the component (E) that has an average primary particle diameter of less than 0.01 µm.

The content of the component (E) is not particularly limited, and is preferably 50 to 500 parts by mass, more preferably 100 to 400 parts by mass, further preferably 125 to 300 parts by mass, and still further preferably 150 to 260 parts by mass, per 100 parts by mass in total of the component (A) and the component (B), from the standpoint of the handleability of the dental curable composition and the strength of the cured product thereof.

The dental curable composition is not particularly limited, as far as containing the component (A), the component (B), the component (C), the component (D), and the component (E), and can be easily produced by a method having been known by a skilled person in the art.

The total amount of the component (A), the component (B), the component (C), the component (D), and the component (E) in the dental curable composition is preferably 60% by mass or more, more preferably 80% by mass or more, further preferably 90% by mass or more, still further preferably 95% by mass or more, and still more further preferably 98% by mass or more, based on 100% by mass of the dental curable composition, from the standpoint of further facilitating the effects of the present invention, and is 100% by mass or less. In other words, in one embodiment of the present invention, the total amount of the component (A), the component (B), the component (C), the component (D), and the component (E) in the dental curable composition is preferably 60 to 100% by mass, more preferably 80 to 100% by mass, further preferably 90 to 100% by mass, still further preferably 95 to 100% by mass, and still more further preferably 98 to 100% by mass, based on 100% by mass of the dental curable composition.

### <Additional Additives>

The dental curable composition may contain known additional additives depending on necessity in such a range in which the effects of the present invention can be exerted. Examples of the additional additives include a photopolymerization initiator, a polymerization inhibitor, an antioxidant, a pigment, a dye, an ultraviolet ray absorbent, an organic solvent, and a thickener.

Examples of the photopolymerization initiator include a (bis)acylphosphine oxide compound, a thioxanthone compound or a quaternary ammonium salt of a thioxanthone compound, a ketal compound, an α-diketone compound, a coumarin compound, an anthraquinone compound, a benzoin alkyl ether compound, and an α-aminoketone based compound.

Among the photopolymerization initiators, at least one kind selected from the group consisting of a (bis)acylphosphine oxide compound and a salt thereof, and an α-diketone compound is preferably used. According to the configuration, a composition that is excellent in photocurability in the visible and near ultraviolet regions, and shows sufficient photocurability with the use of any light source including a halogen lamp, a light emitting diode (LED), and a xenon lamp can be obtained.

As the (bis)acylphosphine oxide compound, 2,4,6-trimethylbenzoyldiphenylphosphine oxide, 2,4,6-trimethylbenzoylmethoxyphenylphosphine oxide, bis(2,4,6-trimethylbenzoyl)phenylphosphine oxide, and 2,4,6-trimethylbenzoylphenylphosphine oxide sodium salt are more preferable.

Examples of the α-ketone compound include diacetyl, dibenzyl, camphorquinone, 2,3-pentadione, 2,3-octadione, 9,10-phenanthrenequinone, 4,4'-oxybenzyl, and acenaphthenequinone. Among these, camphorquinone is preferred from the standpoint of the presence of the maximum absorption wavelength in the visible region.

In the case where the photopolymerization initiator is used, the content of the photopolymerization initiator in the dental curable composition is not particularly limited, and it is preferred that the photopolymerization initiator is contained in an amount of 0.001 to 30 parts by mass per 100 parts by mass in total of the component (A) and the component (B) from the standpoint of the curability of the dental curable composition and the like. In the case where the content of the photopolymerization initiator is 0.001 part by mass or more, the polymerization can be allowed to proceed sufficiently to prevent stickiness. From this standpoint, the content of the photopolymerization initiator is more preferably 0.01 part by mass or more, further preferably 0.05 part by mass or more, and still further preferably 0.08 part by mass or more, per 100 parts by mass in total of the component (A) and the component (B). In the case where the content of the photopolymerization initiator is 30 parts by mass or less, the deposition of the photopolymerization initiator from the dental curable composition can be prevented. From this standpoint, the content of the photopolymerization initiator is more preferably 10 parts by mass or less, further preferably 5 parts by mass or less, and still further preferably 2 parts by mass or less, per 100 parts by mass in total of the component (A) and the component (B). As described above, the lower limit values and the upper limit values described in a stepwise manner can be independently combined. For example, in one embodiment of the present invention, the content of the photopolymerization initiator in the dental curable composition is preferably 0.001 to 30 parts by mass, more preferably 0.01 to 10 parts by mass, further preferably 0.05 to 5 parts by mass, and still further preferably 0.08 to 2 parts by mass, per 100 parts by mass in total of the component (A) and the component (B).

Examples of the polymerization inhibitor include hydroquinone, hydroquinone monomethyl ether, dibutyl hydroquinone, dibutyl hydroquinone monomethyl ether, t-butyl catechol, 2-t-butyl-4,6-dimethylphenol, 2,6-di-t-butylphenol, and 3,5-di-t-butyl-4-hydroxytoluene. In the case where the dental curable composition contains the polymerization inhibitor, the content of the polymerization inhibitor is preferably 0.001 to 1.0 part by mass, more preferably 0.005 to 0.5 part by mass, and further preferably 0.01 to 0.1 part by mass, per 100 parts by mass in total of the component (A) and the component (B).

The dental curable composition has a particularly small polymerization shrinkage stress, and the polymerization shrinkage stress of the dental curable composition measured by the method shown in the examples described later is preferably 7.0 MPa or less, more preferably 6.0 MPa or less, and further preferably 5.0 MPa or less. The lower limit value of the polymerization shrinkage stress is not particularly limited, and may be, for example, 1.0 MPa.

The dental curable composition has a particularly small polymerization shrinkage stress, and is excellent in mechanical strength of the cured product thereof. Therefore, the dental curable composition can be favorably applied to a dental composite resin that can utilize the advantages thereof, and in particular, can be optimally applied to a dental filling and restorative material, a dental cement, and a dental abutment building material.

### Examples

The present invention will be described more specifically with reference to examples below, but the present invention is not limited to the examples.

The components used in Examples and Comparative Examples and abbreviated names thereof will be described below.

### [(Meth)acrylic based Compound (A) having Two or more (Meth)acryloyl Groups in One Molecule]

### <(Meth)acrylic based Compound (a-I) having Urethane Bond>

UDMA: 2,2,4-trimethylhexamethylenebis(2-carbamoyloxyethyl) dimethacrylate (available from Kyoeisha Chemical Co., Ltd.)
U4TH: N,N'-(2,2,4-trimethylhexamethylene)bis[2-(aminocarboxy)propan-1,3-diol] tetramethacrylate (available from Kyoeisha Chemical Co., Ltd.)

### <(Meth)acrylic based Compound (a-II) containing No Urethane Bond>

D2.6E: 2,2-bis(4-methacryloyloxypolyethoxyphenyl)propane (average addition number of ethoxy group: 2.6, available from Shin-Nakamura Chemical Co., Ltd.)
TEGDMA: triethylene glycol dimethacrylate (available from Shin-Nakamura Chemical Co., Ltd.)

### [Mono(meth)acrylate Ester Compound (B)]

### <Mono(meth)acrylate Ester Compound (b-I) represented by General Formula (I)>

EPPMA: ethoxylated o-phenylphenol methacrylate (available from Kyoeisha Chemical Co., Ltd.)
EPPA: ethoxylated o-phenylphenol acrylate (available from Kyoeisha Chemical Co., Ltd.)

### <Mono(meth)acrylate Ester Compound (b-II) represented by General Formula (II)>

POBMA: m-phenoxybenzyl methacrylate (available from Kyoeisha Chemical Co., Ltd.)
POBA: m-phenoxybenzyl acrylate (available from Kyoeisha Chemical Co., Ltd.)

### <Mono(meth)acrylate Ester Compound other than Component (B)>

MDP: 10-methacryloyloxydecyl dihydrogen phosphate
PEM: phenoxyethyl methacrylate (available from Kyoeisha Chemical Co., Ltd.)
IBM: isobornyl methacrylate (available from Kyoeisha Chemical Co., Ltd.)

### [Chemical Polymerization Initiator (C)]

BPO: benzoyl peroxide
THP: 1,1,3,3-tetramethylbutyl hydroperoxide

### [Polymerization Accelerator (D)]

DEPT: N,N-bis(2-hydroxyethyl)-p-toluidine
PTU: 1-(2-pyridyl)-2-thiourea
DMETU: 4,4-dimethylethylenethiourea
VOAA: vanadyl acetylacetonate
CAT: cupric acetate
PDE: ethyl 4-N,N-dimethylaminobenzoate

### [Filler (E)]

Filler (E)-1: 3-methacryloyloxypropyltrimethoxysilane-treated barium glass "SCHOTT (registered trademark) 8235 UF2.0" (available from SCHOTT AG, average particle diameter: 2.0 µm)
Filler (E)-2: 3-methacryloyloxypropyltrimethoxysilane-treated fluoroaluminosilicate glass "SCHOTT (registered trademark) G018-090 UF1.0" (available from SCHOTT AG, average particle diameter: 1.0 µm)
Filler (E)-3: hydrophilic fumed silica "AEROSIL(registered trademark) 380" (available from Nippon Aerosil Co., Ltd., average primary particle diameter: 0.007 µm)

### [Photopolymerization Initiator]

CQ: camphorquinone

### [Polymerization Inhibitor]

BHT: 3,5-di-t-butyl-4-hydroxytoluene

### [Examples 1 to 10 and Comparative Examples 1 to 6]

The components shown in Tables 1 to 3 below were mixed at ordinary temperature (25°C) to prepare pastes of the first material and the second material for Examples and Comparative Examples 1 to 5, resulting in the dental curable compositions of Examples and Comparative Examples. A kneading apparatus used included a polyolefin based resin container including one pair of barrels having the same shape which are arranged in parallel (total capacity: 5 mL, available from MixPac AG, product code: SDL 005-01-SI), a mixer attached to the top end of the container, and an extruding device including one pair of cylindrical extruding members fixed to each other inserted to the container from the rear end of the container (available from MixPac AG, product code: PED 005-01-SI). The same amounts of the pastes were collected by pressing the extruding members. The mixer used included a mixing chip having eight agitation blades (elements) (available from MixPac AG, product code: ML 2.5-08-S).

In Comparative Example 6, a one-component paste was regulated, and a container of "Clearfil Majesty LV (available from Kuraray Noritake Dental Inc., length of cylinder: 7.5 cm, inner diameter of opening: 0.8 cm, both "Clearfil" and "Majesty" are registered trademarks)" was used with a guide chip having a length of 1.5 cm and an inner diameter of the opening of 0.8 mm attached to the top end of the container.

The resulting dental curable compositions were measured and evaluated for the flexural strength, the flexural modulus, and the polymerization shrinkage stress in the following manner.

### <Flexural Test>

Evaluation was performed by a flexural test according to JIS T6511 2009. For Examples and Comparative Examples 1 to 5, the dental curable composition produced was charged in a stainless steel mold (2 mm in vertical length × 2 mm in thickness × 25 mm in horizontal length), which was held in the vertical direction with slide glass under pressure, and the dental curable composition was cured by immersing in a thermostat water bath at 37°C. For Comparative Example 6, the dental curable composition produced was charged in a stainless steel mold (dimension: 2 mm × 2 mm × 25 mm), which was held in the vertical direction with slide glass under pressure, and cured by irradiating with light on both surfaces thereof at 5 points per one surface for 10 seconds per one point with a dental LED light irradiator for polymerization (product name: Pencure 2000, available from J. Morita MFG. Corporation).

The resulting cured products were subjected to a flexural test by using a universal testing machine ("Autograph (registered trademarks) AG-I 100kN", available from Shimadzu Corporation) at a crosshead speed of 2 mm/min, so as to measure the flexural strength and the flexural modulus.

The results are shown in Tables 1 to 3 below. A specimen having a flexural strength of 100 MPa or more and a flexural modulus of 5.0 GPa or more was designated as acceptable.

### <Polymerization Shrinkage Stress>

The dental curable compositions obtained in Examples and Comparative Examples each were charged in a ring mold (formed of stainless steel, 5.5 mm in inner diameter × 0.8 mm in thickness) placed on a glass plate having a thickness of 4.0 mm. The glass plate used had been subjected to a sandblast treatment with alumina powder having a particle diameter of 50 µm. For Examples and Comparative Examples 1 to 5, a stainless steel jig (5 mm in diameter) connected to a shrinkage stress measuring device (available from Shimadzu Corporation, product name: Micro Strain Tester MST-I) was placed on the charged paste to hold the dental curable composition therebetween, and the test was started. At this time, the polymerization shrinkage stress occurring due to the curing through the progress of polymerization reaction of the dental curable composition was measured with the universal testing machine (n = 3).

For Comparative Example 6, a stainless steel jig (5 mm in diameter) connected to a shrinkage stress measuring device (available from Shimadzu Corporation, product name: Micro Strain Tester MST-I) was placed on the charged paste to hold the dental curable composition therebetween, and the paste was cured by irradiating the paste with light through the glass plate with a dental LED light irradiator for polymerization (product name: Pencure 2000, available from J. Morita MFG. Corporation) for 20 seconds. At this time, the polymerization shrinkage stress occurring due to the curing through the progress of polymerization reaction of the dental curable composition caused by the light irradiation was measured with the universal testing machine (n = 3).

The results are shown in Tables 1 to 3 below. The polymerization shrinkage stress is preferably 7.0 MPa or less, more preferably 6.0 MPa or less, and further preferably 5.0 MPa or less.

**Table 1**

| | | | | Example 1 | | | Example 2 | | | Example 3 | | |
|---|---|---|---|---|---|---|---|---|---|---|---|---|
| | | | | 1st material | 2nd material | Mixture | 1st material | 2nd material | Mixture | 1st material | 2nd material | Mixture |
| | (A) | UDMA | (part by mass) | 75 | 75 | 75 | 75 | 75 | 75 | 75 | 75 | 75 |
| | | U4TH | (part by mass) | | | | | | | | | |
| | | D2.6E | (part by mass) | | | | | | | | | |
| | (B) | POBMA | (part by mass) | 25 | 25 | 25 | 25 | 25 | 25 | 25 | 25 | 25 |
| | | POBA | (part by mass) | | | | | | | | | |
| | | EPPMA | (part by mass) | | | | | | | | | |
| | | EPPA | (part by mass) | | | | | | | | | |
| | (C) | BPO | (part by mass) | 1.5 | | 0.75 | | | | | | |
| | | THP | (part by mass) | | | | 2.0 | | 1.0 | 2.5 | | 1.25 |
| | (D) | DEPT | (part by mass) | | 1.5 | 0.75 | | | | | | |
| | | PTU | (part by mass) | | | | | 2.0 | 1.0 | | | |
| Materials | | DMETU | (part by mass) | | | | | | | | 4.0 | 2.0 |
| | | VOAA | (part by mass) | | | | | 0.05 | 0.025 | | | |
| | | CAT | (part by mass) | | | | | | | | 0.005 | 0.0025 |
| | | PDE | (part by mass) | | | | | 0.6 | 0.3 | | 0.4 | 0.2 |
| | (E) | Filler (E)-1 | (part by mass) | 200 | | 100 | 200 | | 100 | 200 | | 100 |
| | | Filler (E)-2 | (part by mass) | | 200 | 100 | | 200 | 100 | | 200 | 100 |
| | | Filler (E)-3 | (part by mass) | 1.0 | 1.0 | 1.0 | 1.0 | 1.0 | 1.0 | 1.0 | 1.0 | 1.0 |
| | | MDP | (part by mass) | | | | | | | | | |
| | | CQ | (part by mass) | | | | 0.3 | | 0.15 | 0.2 | | 0.1 |
| | | BHT | (part by mass) | 0.01 | 0.05 | 0.03 | 0.01 | 0.05 | 0.03 | 0.01 | 0.05 | 0.03 |
| Properties | | Flexural strength | (MPa) | 115 | | | 118 | | | 114 | | |
| | | Flexural modulus | (GPa) | 5.4 | | | 5.6 | | | 5.5 | | |
| | | Polymerization shrinkage stress | (MPa) | 5.6 | | | 4.8 | | | 4.9 | | |

**Table 1 (continued)**

| | | | | Example 4 | | | Example 5 | | |
|---|---|---|---|---|---|---|---|---|---|
| | | | | 1st material | 2nd material | Mixture | 1st material | 2nd material | Mixture |
| | (A) | UDMA | (part by mass) | 60 | 50 | 55 | 75 | 75 | 75 |
| | | U4TH | (part by mass) | 15 | 25 | 20 | | | |
| | | D2.6E | (part by mass) | | | | | | |
| | (B) | POBMA | (part by mass) | | | | | | |
| | | POBA | (part by mass) | 25 | 25 | 25 | | | |
| | | EPPMA | (part by mass) | | | | 25 | 25 | 25 |
| | | EPPA | (part by mass) | | | | | | |
| | (C) | BPO | (part by mass) | | | | | | |
| | | THP | (part by mass) | 2.0 | | 1.0 | 2.0 | | 1.0 |
| | (D) | DEPT | (part by mass) | | | | | | |
| | | PTU | (part by mass) | | 2.0 | 1.0 | | 2.0 | 1.0 |
| | | DMETU | (part by mass) | | | | | | |
| Materials | | VOAA | (part by mass) | | 0.05 | 0.025 | | 0.05 | 0.025 |
| | | CAT | (part by mass) | | | | | | |
| | | PDE | (part by mass) | | 0.6 | 0.3 | | 0.6 | 0.3 |
| | (E) | Filler (E)-1 | (part by mass) | 200 | | 100 | 200 | | 100 |
| | | Filler (E)-2 | (part by mass) | | 200 | 100 | | 200 | 100 |
| | | Filler (E)-3 | (part by mass) | 1.0 | 1.0 | 1.0 | 1.0 | 1.0 | 1.0 |
| | | MDP | (part by mass) | | | | | | |
| | | CQ | (part by mass) | 0.3 | | 0.15 | 0.3 | | 0.15 |
| | | BHT | (part by mass) | 0.01 | 0.05 | 0.03 | 0.01 | 0.05 | 0.03 |
| Properties | | Flexural strength | (MPa) | 118 | | | 117 | | |
| | | Flexural modulus | (GPa) | 5.7 | | | 5.7 | | |
| | | Polymerization shrinkage stress | (MPa) | 5.4 | | | 4.9 | | |

**Table 2**

| | | | | Example 6 | | | Example 7 | | | Example 8 | | |
|---|---|---|---|---|---|---|---|---|---|---|---|---|
| | | | | 1st material | 2nd material | Mixture | 1st material | 2nd material | Mixture | 1st material | 2nd material | Mixture |
| | (A) | UDMA | (part by mass) | 60 | 50 | 55 | 65 | 65 | 65 | 80 | 80 | 80 |
| | | U4TH | (part by mass) | 15 | 25 | 20 | | | | | | |
| | | D2.6E | (part by mass) | | | | | | | | | |
| | (B) | POBMA | (part by mass) | 15 | 15 | 15 | 35 | 35 | 35 | 20 | 20 | 20 |
| | | POBA | (part by mass) | | | | | | | | | |
| | | EPPMA | (part by mass) | | | | | | | | | |
| | | EPPA | (part by mass) | 10 | 10 | 10 | | | | | | |
| | (C) | BPO | (part by mass) | | | | | | | | | |
| | | THP | (part by mass) | 2.0 | | 1.0 | 2.0 | | 1.0 | 2.0 | | 1.0 |
| | (D) | DEPT | (part by mass) | | | | | | | | | |
| | | PTU | (part by mass) | | 2.0 | 1.0 | | 2.0 | 1.0 | | 2.0 | 1.0 |
| Materials | | DMETU | (part by mass) | | | | | | | | | |
| | | VOAA | (part by mass) | | 0.05 | 0.025 | | 0.05 | 0.025 | | 0.05 | 0.025 |
| | | CAT | (part by mass) | | | | | | | | | |
| | | PDE | (part by mass) | | 0.6 | 0.3 | | 0.6 | 0.3 | | 0.6 | 0.3 |
| | (E) | Filler (E)-1 | (part by mass) | 200 | | 100 | 250 | | 125 | 150 | | 75 |
| | | Filler (E)-2 | (part by mass) | | 200 | 100 | | 250 | 125 | | 150 | 75 |
| | | Filler (E)-3 | (part by mass) | 1.0 | 1.0 | 1.0 | 1.0 | 1.0 | 1.0 | 3.0 | 3.0 | 3.0 |
| | | MDP | (part by mass) | | | | | | | | | |
| | | CQ | (part by mass) | 0.3 | | 0.15 | 0.3 | | 0.15 | 0.3 | | 0.15 |
| | | BHT | (part by mass) | 0.01 | 0.05 | 0.03 | 0.01 | 0.05 | 0.03 | 0.01 | 0.05 | 0.03 |
| Properties | | Flexural strength | (MPa) | 117 | | | 124 | | | 127 | | |
| | | Flexural modulus | (GPa) | 5.7 | | | 6.2 | | | 5.3 | | |
| | | Polymerization shrinkage stress | (MPa) | 5.3 | | | 4.9 | | | 4.8 | | |

**Table 2 (continued)**

| | | | | Example 9 | | | Example 10 | | |
|---|---|---|---|---|---|---|---|---|---|
| | | | | 1st material | 2nd material | Mixture | 1st material | 2nd material | Mixture |
| Materials | (A) | UDMA | (part by mass) | | | | 75 | 75 | 75 |
| | | U4TH | (part by mass) | | | | | | |
| | | D2.6E | (part by mass) | 75 | 75 | 75 | | | |
| | (B) | POBMA | (part by mass) | 25 | 25 | 25 | 25 | 25 | 25 |
| | | POBA | (part by mass) | | | | | | |
| | | EPPMA | (part by mass) | | | | | | |
| | | EPPA | (part by mass) | | | | | | |
| | (C) | BPO | (part by mass) | | | | 1.5 | | 0.75 |
| | | THP | (part by mass) | 2.0 | | 1.0 | | | |
| | (D) | DEPT | (part by mass) | | | | | 1.5 | 0.75 |
| | | PTU | (part by mass) | | 2.0 | 1.0 | | | |
| | | DMETU | (part by mass) | | | | | | |
| | | VOAA | (part by mass) | | 0.05 | 0.025 | | | |
| | | CAT | (part by mass) | | | | | | |
| | | PDE | (part by mass) | | 0.6 | 0.3 | | 0.6 | 0.3 |
| | (E) | Filler (E)-1 | (part by mass) | 200 | | 100 | 200 | | 100 |
| | | Filler (E)-2 | (part by mass) | | 200 | 100 | | 200 | 100 |
| | | Filler (E)-3 | (part by mass) | 1.0 | 1.0 | 1.0 | 1.0 | 1.0 | 1.0 |
| | | MDP | (part by mass) | | | | 5.0 | | 2.5 |
| | | CQ | (part by mass) | 0.3 | | 0.15 | 0.3 | | 0.15 |
| | | BHT | (part by mass) | 0.01 | 0.05 | 0.03 | 0.01 | 0.05 | 0.03 |
| Properties | | Flexural strength | (MPa) | 106 | | | 102 | | |
| | | Flexural modulus | (GPa) | 5.2 | | | 5.1 | | |
| | | Polymerization shrinkage stress | (MPa) | 6.7 | | | 4.8 | | |

**Table 3**

| | | | | Comparative Example 1 | | | Comparative Example 2 | | | Comparative Example 3 | | |
|---|---|---|---|---|---|---|---|---|---|---|---|---|
| | | | | 1st material | 2nd material | Mixture | 1st material | 2nd material | Mixture | 1st material | 2nd material | Mixture |
| | (A) | UDMA | (part by mass) | 75 | 75 | 75 | | | | 75 | 75 | 75 |
| | | D2.6E | (part by mass) | | | | 75 | 75 | 75 | | | |
| | | TEGDMA | (part by mass) | 25 | 25 | 25 | 25 | 25 | 25 | | | |
| | (B) | POBMA | (part by mass) | | | | | | | | | |
| | (C) | THP | (part by mass) | 2.0 | | 1.0 | 2.0 | | 1.0 | 2.0 | | 1.0 |
| | (D) | PTU | (part by mass) | | 2.0 | 1.0 | | 2.0 | 1.0 | | 2.0 | 1.0 |
| Materials | | VOAA | (part by mass) | | 0.05 | 0.025 | | 0.05 | 0.025 | | 0.05 | 0.025 |
| | | PDE | (part by mass) | | 0.6 | 0.3 | | 0.6 | 0.3 | | 0.6 | 0.3 |
| | (E) | Filler (E)-1 | (part by mass) | 200 | | 100 | 200 | | 100 | 200 | | 100 |
| | | Filler (E)-2 | (part by mass) | | 200 | 100 | | 200 | 100 | | 200 | 100 |
| | | Filler (E)-3 | (part by mass) | 1.0 | 1.0 | 1.0 | 1.0 | 1.0 | 1.0 | 1.0 | 1.0 | 1.0 |
| | | PEM | (part by mass) | | | | | | | 25 | 25 | 25 |
| | | IBM | (part by mass) | | | | | | | | | |
| | | CQ | (part by mass) | 0.3 | | 0.15 | 0.3 | | 0.15 | 0.3 | | 0.15 |
| | | BHT | (part by mass) | 0.01 | 0.05 | 0.03 | 0.01 | 0.05 | 0.03 | 0.01 | 0.05 | 0.03 |
| properties | | Flexural strength | (MPa) | 119 | | | 126 | | | 82 | | |
| | | Flexural modulus | (GPa) | 5.7 | | | 5.9 | | | 3.8 | | |
| | | Polymerization shrinkage stress | (MPa) | 8.9 | | | 9.6 | | | 7.6 | | |

**Table 3 (continued)**

| | | | | Comparative Example 4 | | | Comparative Example 5 | | | Comparative Example 6 |
|---|---|---|---|---|---|---|---|---|---|---|
| | | | | 1st material | 2nd material | Mixture | 1st material | 2nd material | Mixture | One-component type |
| | (A) | UDMA | (part by mass) | 75 | 75 | 75 | | | | 75 |
| | | D2.6E | (part by mass) | | | | | | | |
| | | TEGDMA | (part by mass) | | | | | | | |
| | (B) | POBMA | (part by mass) | | | | 100 | 100 | 100 | 25 |
| | (C) | THP | (part by mass) | 2.0 | | 1.0 | 2.0 | | 1.0 | |
| | (D) | PTU | (part by mass) | | 2.0 | 1.0 | | | | |
| | | VOAA | (part by mass) | | 0.05 | 0.025 | | 0.05 | 0.025 | |
| | | PDE | (part by mass) | | 0.6 | 0.3 | | 0.6 | 0.3 | 0.6 |
| Materials | (E) | Filler (E)-1 | (part by mass) | 200 | | 100 | 200 | | 100 | 200 |
| | | Filler (E)-2 | (part by mass) | | 200 | 100 | | 200 | 100 | |
| | | Filler (E)-3 | (part by mass) | 1.0 | 1.0 | 1.0 | 1.0 | 1.0 | 1.0 | 1.0 |
| | | PEM | (part by mass) | | | | | | | |
| | | IBM | (part by mass) | 25 | 25 | 25 | | | | |
| | | CQ | (part by mass) | 0.3 | | 0.15 | 0.3 | | 0.15 | 0.30 |
| | | BHT | (part by mass) | 0.01 | 0.05 | 0.03 | 0.01 | 0.05 | 0.03 | 0.01 |
| properties | | Flexural strength | (MPa) | 117 | | | 31 | | | 108 |
| | | Flexural modulus | (GPa) | 5.5 | | | 2.4 | | | 5.2 |
| | | Polymerization shrinkage stress | (MPa) | 8.4 | | | 1.7 | | | 7.8 |

It is understood from the results in Tables 1 and 2 that the two-component type dental curable compositions of Examples 1 to 10 containing the components (A) to (E) are excellent due to the appropriate flexural strength, the appropriate flexural modulus, and the small polymerization shrinkage stress in curing.

On the other hand, it is understood from the results in Table 3 that the two-component type dental curable compositions of Comparative Examples 1 to 4 containing no component (B) have a large polymerization shrinkage stress. It is also understood therefrom that the two-component type dental curable composition of Comparative Example 5 containing no component (A) has a low flexural strength and a low flexural modulus. It is also understood therefrom that the one-component type dental curable composition of Comparative Example 6 containing no component (C) has a large polymerization shrinkage stress.

### Industrial Applicability

The dental curable composition can be favorably applied to a dental composite resin, and particularly to a dental filling and restorative material, a dental cement, and a dental abutment building material.

## Claims

1. A dental curable composition comprising a (meth)acrylic based compound (A) having two or more (meth)acryloyl groups in one molecule, a mono(meth)acrylate ester compound (B), a chemical polymerization initiator (C), a polymerization accelerator (D), and a filler (E), the mono(meth)acrylate ester compound (B) containing at least one kind selected from the group consisting of a mono(meth)acrylate ester compound (b-I) represented by the following general formula (I) and a mono(meth)acrylate ester compound (b-II) represented by the following general formula (II), the dental curable composition being separately packaged in a first material containing the chemical polymerization initiator (C) and a second material containing the polymerization accelerator (D): 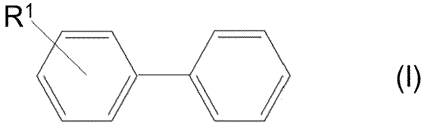 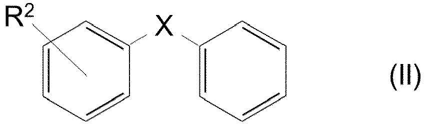 wherein in the general formulae (I) and (II), R¹ and R² each independently represent a group represented by the following general formula (i) or a group represented by the following general formula (ii), and X represents a divalent hydrocarbon group having 1 to 6 carbon atoms or an oxygen atom, 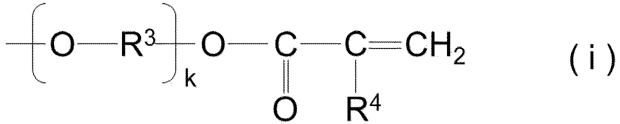 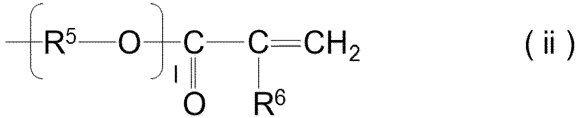 wherein in the general formulae (i) and (ii), R³ and R⁵ each independently represent a divalent hydrocarbon group having 1 to 10 carbon atoms, R⁴ and R⁶ each independently represent a hydrogen atom or a methyl group, and k and l each independently represent an integer of 0 to 6.

2. The dental curable composition according to claim 1, wherein R⁴ and R⁶ each represent a methyl group.

3. The dental curable composition according to claim 1 or 2, wherein k and l each are 0 or 1.

4. The dental curable composition according to any one of claims 1 to 3, wherein the component (B) contains the compound (b-II), and X represents an oxygen atom.

5. The dental curable composition according to any one of claims 1 to 4, wherein R² represents a group represented by the general formula (ii).

6. The dental curable composition according to any one of claims 1 to 5, wherein the component (A) contains a (meth)acrylic based compound (a-I) containing a urethane bond.

7. A dental filling and restorative material comprising the dental curable composition according to any one of claims 1 to 6.

8. A dental cement comprising the dental curable composition according to any one of claims 1 to 6.

9. A dental abutment building material comprising the dental curable composition according to any one of claims 1 to 6.
